# EUROPEAN PATENT SPECIFICATION

(11) **EP 3 645 124 B1**
(45) Date of publication and mention of the grant of the patent: **10.07.2024**
(21) Application number: 18738218.9
(22) Date of filing: 26.06.2018
(51) Int. Cl.: A61Q 19/00, A61Q 19/02, A61Q 19/08, A61K 8/02, A61K 8/25, A61K 8/26, A61K 8/27, A61K 8/29, C30B 29/60, C30B 29/16, C04B 38/06, B82Y 20/00, B82Y 5/00, B82Y 30/00, C01G 1/02, C04B 38/00, C30B 5/00, C01G 23/053

(54) **COSMETIC COMPOSITION COMPRISING AN ORDERED POROUS MATERIAL FOR REDUCING THE VISIBLE AND/OR TACTILE IRREGULARITIES OF THE SKIN**
KOSMETISCHE ZUSAMMENSETZUNG MIT GEORDNETEM PORÖSEM MATERIAL ZUR REDUZIERUNG DER SICHTBAREN UND/ODER TAKTILEN UNREGELMÄSSIGKEITEN VON DER HAUT
COMPOSITION COSMÉTIQUE COMPRENANT UNE MATIÈRE POREUSE ORDONNÉE POUR RÉDUIRE LES IRRÉGULARITÉS VISIBLES ET/OU TACTILES DE LA PEAU

(30) Priority: 26.06.2017 EP 17305792
(43) Date of publication of application: 06.05.2020
(73) Proprietor: L'OREAL, 75008 Paris (FR)
(72) Inventor: VIDAL, Laurent, 93600 Aulnay Sous Bois (FR); BESNADIERE, Julie, 93600 Aulnay Sous Bois (FR)
(74) Representative: Cabinet Nony
(86) International application number: PCT/EP2018/067083
(87) International publication number: WO 2019/002275

(56) References cited:
- WO-A1-2016/198527
- WO-A1-2016/198537
- WO-A2-2008/079543
- US-B1- 6 680 013
- SCHRODEN R C ET AL: "OPTICAL PROPERTIES OF INVERSE OPAL PHOTONIC CRYSTALS", CHEMISTRY OF MATERIALS, AMERICAN CHEMICAL SOCIETY, US, vol. 14, no. 8, 1 August 2002 (2002-08-01), pages 3305 - 3315, XP001164086, ISSN: 0897-4756, DOI: 10.1021/CM020100Z
- SCHRODEN RICK C ET AL: "Inverse Opal Photonic Crystal A Laboratory Guide", pages 1 - 32, XP002503027, Retrieved from the Internet <URL:http://www.mrsec.umn.edu/ehr/InverseOpal.Guide.pdf> [retrieved on 20081106]
- CARLOS I AGUIRRE ET AL: "Tunable Colors in Opals and Inverse Opal Photonic Crystals", ADVANCED FUNCTIONAL MATERIALS, WILEY - V C H VERLAG GMBH & CO. KGAA, DE, vol. 20, no. 16, 23 August 2010 (2010-08-23), pages 2565 - 2578, XP001556985, ISSN: 1616-301X, [retrieved on 20100720], DOI: 10.1002/ADFM.201000143
- DATABASE WPI Week 201444, Derwent World Patents Index; AN 2014-M50423, XP002773309

## Description

### TECHNICAL FIELD

The present invention relates to a cosmetic method for improving the surface appearance of skin having relief, coloration irregularities or aging related irregularities or defects, and more particularly visible and/or tactile irregularities.

The present invention also relates to a novel inorganic material comprising particles of three-dimensional ordered porous structure comprising spheroidal pores. The present invention also relates to a cosmetic composition comprising these particles. These compositions are referred to as compositions having improved soft focus effect properties.

### BACKGROUND OF THE INVENTION

It is common to observe that people with skin imperfections and irregularities related to helioderma, acne scars or to aging, such as wrinkles, fine lines, dilated pores, hyperkeratinization, wish to mask these defects and use for this purpose cosmetic or dermatological compositions or anti-ageing protocols.

These people thus may use cosmetic compositions making it possible to reduce the consequences of the hypersecretion of sebum via the pores, or making it possible to exfoliate the dead cells of the epidermis or to induce a peeling effect intended to destroy in a controlled manner the damaged epidermis or the superficial layers of the dermis. These people await a regeneration of the destroyed layers and a reproduction of the elastic fibers and collagen in the underlying layers in order to densify the skin. For example, these compositions contain derivatives of AHA such as glycolic acid or trochloracetic acid.

Unfortunately, the obtained effects are not immediate and their efficacy is relatively insufficient and in some cases undesirable reactions of cutaneous intolerance occur.

Another solution consists in using protocols such as the injection of botulinum toxin (Botox) to fight known muscle hypertonia or hyaluronic acid to compensate for the loss of volume. These injections are carried out by specialists and induce clearly visible effects on the perception of irregularities. On the other hand, these protocols are long, invasive, and do not allow an immediate effect and are not applicable by the patients themselves.

Another solution is to use thermating, which is a protocol using radiofrequencies to thermally stimulate the production of collagen and to favor the tightening of elastin fibers. Again, it is performed under medical supervision, not allowing immediate and self-enforcing effects.

Those wishing to limit the visibility of their wrinkles may also think of using cosmetic compositions containing retinoids such as retinol to stimulate the production of collagen. Other cosmetic compositions contain agents acting on the growth factor or else vitamin C. The effect is however obtained only after 6 to 9 weeks.

In the field of cosmetic compositions providing a rapidly perceived effect, silicone derivatives whose mode of action consists in bridging the zones excavated by the pores and the fine lines have already been used. The perception of irregularities of relief is thus diminished without, however, offering an important and transparent masking.

In order to avoid the use of cosmetic compositions which impart a very dull appearance of the skin and which thus contribute to a perception of artificially aged skin, cosmetic compositions containing moisturizing agents make it possible to combat the excess visibility of wrinkles due to the drying of the skin.

Another solution consists in using cosmetic compositions such as foundations imparting coverage of fine wrinkles. The effect is immediate but the coverage thus provided confers on the face an unnatural appearance and an undesirable mask effect for people seeking to preserve the natural appearance of their face or more generally of their skin.

Many cosmetic compositions have been developed for the purpose of attempting to hide skin defects or imperfections such as, for example, wrinkles, fine lines, pores, roughness, etc. Such attempts have included incorporating ingredients into the compositions which reflect and/or diffuse light to de-emphasize the defects and imperfections under the form of micro-particles. Emmert, "Quantification of the Soft-Focus Effect," Cosmetics and Toiletries, 111, 57-61 (1996) discusses attempting to create a soft-focus effect using such light-diffusing/scattering ingredients. When soft focus index is high, a composition produces a large soft-focus effect, serving to mask defects by changing the perception of relief.

The particles must have (1) a minimum light absorption in the visible region, (2) a high total transmission in order not to create a natural appearance of the skin, (3) this transmission being diffused in such a way that the backscatter produced by reflection of the rays by the skin appears to be evenly distributed, (4) a minimum specular reflection do as to minimize luster that would increase the appearance of wrinkles, and (5) a reflection by high scattering so as to have an even light distribution over the area independent of underlying wrinkles.

Usually, such cosmetic compositions contain either boron nitride particles or nylon particles, polymer-coated aluminum plates, spherical silica particles, silicone elastomers, planar powders, spherical powders such as PMMA, barium sulfate, and/or powders with a high refractive index such as titanium dioxide, zinc oxide and iron oxide in an attempt to achieve a soft-focus effect. However, such products have had drawbacks such as, for example, an unnatural look on skin owing to the use of highly opaque materials and the highlighting of skin defects owing to the use of planar materials. Moreover, such products have not generally had optimal or desirable soft-focus effect properties.

Therefore, there is consequently a need to provide new cosmetic methods conferring a masking of irregularities of skins having relief, coloration irregularities, for example spots or aging related irregularities, in an immediate, durable manner, including at the level of fine wrinkles and more visible wrinkles such as expression lines, while maintaining a very natural appearance of the skin without covering marks.

In particular, there is a real need for materials contained in cosmetic compositions, such as they confer a very blurry appearance and actually reduce the visibility of skin having relief, coloration irregularities or aging related irregularities, in a durable manner, including also while being transparent in the visible, colorless or colored, to ensure a natural, non-opaque or covering appearance when applied to skins having relief, coloration irregularities, such as skin spots or aging related irregularities.

There is also a need to obtain cosmetic films on skins having such irregularities defects or imperfections, which are perfectly transparent in the visible range or which can impart a natural color while being transparent.

There also remains a need for suitable cosmetic method implementing cosmetic compositions having improved soft focus effect properties.

The object of the present invention is, precisely, to satisfy all or some of the needs by overcoming the drawbacks mentioned above.

### SUMMARY OF THE INVENTION

Unexpectedly, and advantageously, the inventors of the present application have found that these disadvantages could be overcome by means of inorganic material comprising particles of three-dimensional ordered porous structure comprising spheroidal pores and compositions containing them.

The inventors have particularly demonstrated, as illustrated in the examples, that the claimed material presents the capacity to improve the surface appearance of skin having relief, skin spots or aging related irregularities, by their transparency.

More specifically, the inventors surprisingly discovered that inorganic materials comprising a continuous phase being obtained from (c) a network of covalently bonded metal oxide and metalloid as recited in the claims, when implemented in cosmetic compositions applied on skin, are able to produce a natural effect on the skin, in particular by diminishing the contrasts through an optic phenomenon. Such inorganic materials, in said environment allow back-scattering of a minimal amount of visible light, and the transmission of the right amount of it to produce such camouflaging effect.

According to one of its first aspects, one subject of the present invention relates to a cosmetic method for improving the surface appearance of skin having relief, skin having spots or aging related irregularities, comprising applying to human skin in need thereof a composition comprising an inorganic material made of particles having a three-dimensional ordered porous structure comprising spheroidal pores,
- said pores having an average pore diameter ranging from 50 nm to 500 nm,
- the pore diameter varying by no greater than 15%,
- said particles having an average largest dimension ranging from 1 to 50 µm, and wherein
said inorganic material comprising a continuous phase obtained from (c) a network of covalently bonded metal oxide and metalloid, the metal oxide and the metalloid being of (a) at least a metal oxide of metal having a valency between 1 and 6 and (b) at least SiO₂ or SiₓO_{y} with x and y comprised independently from another between 0.1 and 2.

In particular, the present invention relates to a cosmetic method for improving the surface appearance of skin having relief, by applying to human skin in need thereof a composition as described above, preferably having improved soft focus effect properties.

In particular, the present invention relates to improving the surface appearance of skin having at least one skin relief imperfection or irregularity, in particular related to helioderma, acne scars or to aging, such as fine wrinkles, fine lines, dilated pores, hyperkeratinisation.

The cosmetic method according to the invention is particularly suitable for application to human skin of individuals with skin imperfections and irregularities related to helioderma, acne scars or to aging, such as fine wrinkles, fine lines, dilated pores, hyperkeratinisation, who wish to mask these defects.

Another subject of the invention is an inorganic material comprising particles having a three-dimensional ordered macroporous structure comprising spheroidal pores,
- said pores having an average pore diameter being greater than 280 nm and less than 500 nm,
- the pore diameter varying by no greater than 15%,
- said particles having an average largest dimension ranging from 1 to 50 µm, and wherein
said inorganic material comprising a continuous phase made of a network of covalently bonded metal oxide and metalloid, the metal oxide and the metalloid being of:
(a) at least a metal oxide of metal M having a valency between 1 and 6, and
(b) at least SiO₂ or SiₓO_{y} with x and y comprised independently from another between 0.1 and 2, in a weight ratio (a)/(b) molar ratio between Si and M as referred to in (a) comprised between 5/95 and 80/20.

According to another of its aspects, a subject of the present invention is a process for manufacturing an inorganic material comprising particles having a three-dimension ordered porous structure comprising a solid continuous phase and spheroidal pores comprising the steps of:
(i) providing a colloidal crystal formed of a regular array of monodisperse organic polymer particles as a template, the diameter of said organic polymer particles varying between 65 and 650 nm,
(ii) adding a precursor of the continuous phase, so as to produce an amorphous solid into the interstices between the monodisperse particles of the colloidal crystal and to form said solid continuous phase and a composite colloidal crystal thereof, and
(iii) removing the monodisperse particles so as to form a regular array of pores in said solid continuous phase,
   wherein said precursor of said continuous phase comprises a mixture of
   (a) at least a precursor of metal oxide of metal having a valency between 1 and 6, and
   (b) at least a precursor of SiO₂ or SiₓO_{y} with x and y comprised independently from another between 0.1 and 2, in a weight ratio (a)/(b) molar ratio between Si and M as referred to in (a) comprised between 5/95 and 80/20.

The present invention further relates to methods of camouflaging and/or disguising skin imperfections comprising applying, in particular a skin imperfection camouflaging and/or disguising effective amount, of a composition comprising an inorganic material as described or claimed in the present invention, to skin in need of such camouflaging or disguising.

The present invention further relates to methods of enhancing the appearance of aged skin or skin having relief or skin spots, in particular irregularities related to helioderma, acne scars, fine lines, dilated pores or hyperkeratinization, by applying compositions comprising an inorganic material as described or claimed in the present invention to the keratinous material, in particular in an amount sufficient to enhance the appearance of the keratinous material.

### FIGURES

- Figure 1 represents a SEM image of the material M1 with a continuous phase made of 5% TiO₂ - 95% SiO₂ as prepared in example 1.
- Figure 2 represents a SEM image of the material M2 with a continuous phase made of 25% TiO₂ - 75% SiO₂ as prepared in example 1.
- Figure 3 represents a SEM image of the material M3 with a continuous phase made of 50% TiO₂ - 50% SiO₂ as prepared in example 1.
- Figure 4 represents a SEM image of the material M4 with a continuous phase made of 75% TiO₂ -25% SiO₂ as prepared in example 1.
- Figure 5 represents a SEM image of the material M5 with a continuous phase made of 100% SiO₂.
- Figure 6 represents a SEM image of the material M6 with a continuous phase made of 100% TiO₂ as prepared in example 1.
- Figure 7 represents a SEM image of the material M7 with a continuous phase made of 100% TiO₂ as prepared in example 1.
- Figure 8 is a scheme of the pores/continuous phase arrangement.

### DESCRIPTION OF THE PREFERRED EMBODIMENTS

In the framework of the present invention the following terms
- "three-dimensionally ordered porous structure" means a structure where the pores are arranged or form a regular periodic array within a solid continuous phase
- For the purposes of the present invention, the term "skin" is intended to denote all of the skin of the body, including the lips, and preferably the skin of the face, the neck and the neckline, and also the lips.
- The term an "aged skin" is intended to denote a skin showing signs of aging, such as wrinkles.
- The term "skin having relief or coloration irregularities" is related to denote a skin having irregularities or skin spots, in particular related to helioderma, acne scars, fine lines, dilated pores or hyperkeratinization.
- The term "room temperature" is intended to denote a temperature of about 25°C. It is set at atmospheric pressure (i.e. a pressure of 1.013 × 10⁵ Pa),
- The "refractive index" is related to the interaction between a material and a propagating radiation within the material. Said refractive index cannot be measured directly. It can be calculated according to the formulae described in the publication of A. Stein and al., Materials Views, 2010, 20, 2565-2578.
- Haze was measured by a method consisting in depositing a composition according to the invention on a polyester film sold under the reference 100045425 by the society BYK using a tire-film applicator to apply a 50 µm thick layer that was dried for 30 minutes at room temperature (25°C). On the obtained dry layer, haze and transparency measurements were performed, using a Hazegard plus apparatus from BYC Additive & Instruments.

### PARTICLES

The inorganic material according to the present invention comprises particles of a three-dimensionally ordered macroporous structure comprising a solid phase with spheroidal pores.

The particles are more particularly detailed hereinafter.

As a first remark, it may be noticed that the present inorganic material is not dedicated for providing color, but rather a slightly white color.

The inorganic material implemented with a cosmetic method, object of the present invention comprises a continuous phase, said inorganic material comprising a continuous phase being obtained from (c) a network of covalently bonded metal oxide and metalloid, the metal oxide and the metalloid being of (a) at least a metal oxide of metal having a valency between 1 and 6 and (b) at least SiO2 or SixOy with x and y comprised independently from another between 0.1 and 2. Said inorganic material will be considered as pertaining to the "*first embodiment*" in the following description.

Furthermore, an inorganic material object of the present invention comprises a continuous phase made of a network of covalently bonded metal oxide and metalloid, the metal oxide and the metalloid being of:
(a) at least a metal oxide of metal having a valency between 1 and 6, and
(b) at least SiO₂ or SiₓO_{y} with x and y comprised independently from another between 0.1 and 2, in a molar ratio between Si and M as referred to in (a) comprised between 5/95 and 80/20. Said inorganic material will be considered as pertaining to the ***"second embodiment"*** in the following description.

In other words, said new material of said second embodiment consists in an entangled inorganic network of Si-O-M, where M is a metal of a valency between 1 and 6 in such a three-dimensional ordered macroporous structure comprising spheroidal pores.

Preferably, the inorganic material according to the first and second embodiments, and in particular according to the second embodiment, does not provide colour.

### Particle size

Said particles have an average largest dimension for both embodiments as described above, also called size in the present invention ranging from 1 to 50 µm,

Said size advantageously ranges from 1 to 20 µm, and in particular from 5 to 10 µm.

The particles according to the present invention have a polymorphic shape and preferably are under the form of platelets.

### Pores

Depending on the chemical nature of the inorganic material of the particles, the pore diameter d is calculated according to the physical rules established in the literature ("Optical Properties of Inverse Opal Photonic Crystals" Schroden and al, Chem. Mater. 2002, 14, 3305-3315) so as to predetermining the range of visible or UV wavelength the particles should reflect. A range of monodisperse organic polymer spheres, useful to form a template, as will be more apparent from the description herein after, is defined from this criterion with different diameters, these diameters being within an interval [d; 1,3.d].

The pores in the inorganic material present a spheroidal shape. The pores are arranged in the inorganic structure so as to have a substantially regular or constant spacing there between. However, as the skilled person could appreciate, some defects in the macroporous structure are unavoidable.

The "average pore diameter" means the mean pore diameter. It may be measured by Scanning Electron Microscopy (SEM) for instance.

The average pore diameter for the first embodiment as described above ranges from 50 to 500 nm, in particular from 120 nm to 380 nm, and more particularly from 150 nm to 280 nm.

The average pore diameter for the second embodiment as described above ranges from 280 to 500 nm, in particular from 280 nm to 380 nm.

In the framework of the present invention, the term "greater than" does not encompass the lower limit value.

In other words, the present invention relates to a "uniform" porous structure, i.e. encompassing pores that have substantially the same diameter. This means that the pore diameter in the framework of the present invention is preferably monodisperse or else that the pore diameter varies by no greater than 15%, preferably than 10% and even more preferably than 5%. This further means that the difference between the pore diameter value of any two particles taken from the whole particles according to the invention does not exceed 15% with respect to the largest pore diameter of said two particles.

### Inorganic material / Continuous phase

The following explanation concerns both embodiments as described above.

The metal oxide of the inorganic material conform to the present invention is selected from ZnO, TiO₂, Fe₂O₃, Al₂O₃, or a mixture thereof, and is more particularly TiO₂.

According to a particular embodiment, the continuous phase is obtained from a mixture (c) of (a) TiO₂ and (b) SiO₂ or SiₓO_{y} with x and y comprised independently from another between 0.1 and 2. According to a particularly preferred embodiment, the continuous phase is obtained from a mixture of TiO₂ and SiO₂ or SiₓO_{y} in a weight ratio between 5/95 and 95/5, preferably between 25/75 and 75/25.

Without wishing to be bound by any theory, the inventors have noted that compositions comprising inorganic material with a continuous phase obtained with a mixture (c) of a metal oxide (a) and SiO₂ or SiₓO_{y} present particularly good soft-effect properties. Indeed, as demonstrated in the examples below, such particles confer an excellent transparency and an excellent haze power.

The solid continuous phase for both embodiments, may be present in an amount from 1 to 26% relative to the total volume of the materials, preferably from 3 to 10%.

The pores preferably contain air.

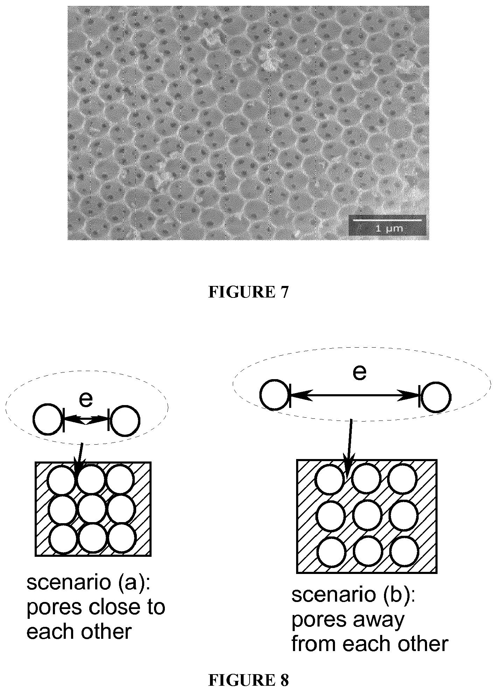

Figure 8 illustrates two particular arrangements cases which are both encompassed within the framework of the present invention. According to the invention the pores are perfectly organized in the material and follow a regular periodical arrangement in it. The distance between each pore which is represented by the parameter e in Figure 8 is constant and varies from 2 to 100 nm, preferably between 2 to 20 nm and even more preferably between 5 to 10 nm.

During the formation of the solid continuous phase according to the invention and as it will be further described below, the formed colloidal crystal composed of polymeric particles of a size that is determined in order to further obtain a desired size of pores in the material, is covered by a solution of a combination of metal oxide precursors.

The particles have preferably a full volume fraction calculated from 1% to 26 %.

The method of calculation of full volume fraction is disclosed in the article "Tuning solvent-dependent color changes of three-Dimensionally ordered porous (3DOM) materials through compositional and geometric modifications" Blanford and al, Advanced Materials, 2001,13, 1, 26-29.

As will the apparent form the following paragraphs related to the manufacturing process, and as already mentioned above, the formation of the pores may be performed thanks to organic particles as a template. Such particles may be extracted by a calcination process.

### Manufacturing process

According to the present invention, the manufacturing process implements organic polymer templates for the formation of the pores within the inorganic structure.

Such a process is for example described in US 6, 680,013 and J. Polym. Chem, 1992, 30, 137. Document WO2016/198537 also described the manufacturing process of some ordered macroporous materials suitable for the present invention, in particular for the first embodiment as described above.

Said process relates to the formation of the inorganic material / Continuous phase described above, i.e. the inorganic material implemented in the cosmetic method subject matter of the present invention (first embodiment as defined above) and the new organic material as a further subject matter of the present invention (second embodiment as defined above).

A process for manufacturing an inorganic material according to the present invention may comprise the steps of:
(i) providing a colloidal crystal formed of a regular array of monodisperse organic polymer particles as a template, the diameter of said organic polymer particles varying between 65 and 650 nm,
(ii) adding a precursor of the continuous phase, so as to produce a solid into the interstices between the monodisperse particles of the colloidal crystal and to form said solid continuous phase and a composite colloidal crystal thereof, and
(iii) removing the monodisperse particles so as to form a regular array of pores in said solid continuous phase.

Each of said steps may be performed according to known methods.

Step (i), (ii) and (iii) are more particularly detailed herein below.

### STEP (i); formation of the colloidal crystal

The colloidal crystal can be formed from monodisperse organic particles as a template.

Such monodisperse particles are commercially available or can be prepared by methods known in the art.

Monodisperse particles made from organic polymer particles may be prepared as dispersions using emulsion, dispersion or suspension polymerization.

Suitable hydrophobic monomers include styrenics, acrylonitrile, methacrylonitrile, acrylates, methacrylates, methacryl amides, acrylamides, maleimides, vinyl ethers, vinyl esters, monoalkylmaleates, dialkyl maleates, fluorinated acrylates and fluorinated methacrylates.

Examples of preferred organic polymer particles include polystyrene spheres, polymethyl methacrylate (PMMA) spheres, Such particles are commercially available from sources such as Polysciences, Inc., Interfacial Dynamics Corp., and Duke Scientific Corp.

Monodispersed poly(methylmethacrulate) or PMMA may in particular be used as a template.

PMMA colloids may alternatively be prepared from monomer methylmethacrylate according to the protocol described in the literature ("Model Filled Polymers. V. Synthesis of Crosslinked Monodisperse Polymethacrylate Beads", Zou and al, J. Polym Sci Part A Polym Chem 1992, 30, 137). They can as well be prepared according to another procedure in which they add a stabilizer to the methylmethacrylate monomer ("Preparation of monodisperse PMMA microspheres in nonpolar solvents by dispersion polymerization with a macromonomeric stabilizer", Klein et al., Colloid Polym Sci 2003, 282: 7-13).

To ensure excellent monodispersity, the polymerization temperature may be set between 45 and 120°C, in particular between 60 and 100°C, and for example at 80 °C. The monomer concentration may be between 1 and 2 mol L⁻¹, preferably between 1.3 and 1.8 mol L⁻¹.

According to the first embodiment, the size of organic polymer sphere ranges from 100 nm to 650 nm and may in particular range between 180 nm and 500 nm, more preferably between 180 and 380 nm.

According to the second embodiment, the size of organic polymer sphere ranges from 380 nm to 650 nm and may in particular range between 380 nm and 500 nm.

The colloidal crystal template formed during this step (i) preferably is a close-packed array of spheres. As used herein, a close-packed array is an array in which the spheres are packed in the smallest volume possible without significantly distorting the shapes of the spheres. The colloidal crystal template can include defects (e.g., point, line, plane defects), typically in a relatively small number, if desired. The formation of such a template can be done by a variety of methods, such as centrifugation, sedimentation, spin coating, evaporation methods, layer-by-layer growth, crystallization in capillaries, deposition in lithographic patterns.

According to a particular embodiment, the colloidal crystal template is formed by centrifugation. In particular such a technique is efficient as allowing removing solvent from the space between spheres and allowing close-packing.

According to a particular embodiment, the step (i) comprises the formation of a colloidal crystal by centrifugation of a PMMA colloid suspension. Centrifugation speed may then be set between 500 tr.min⁻¹ and 4000 tr.min⁻¹, preferably between 800 tr.min⁻¹ and 3000 tr.min⁻¹. Centrifugation time may vary from 1 hour to 96 hours, preferably between 6 and 72 hours. The supernatant may then be removed and the colloidal crystal thus obtained may be placed in an oven at a temperature between 30 and 60 °C to obtain PMMA spheres ordered material.

The obtained colloidal crystal may then be dried for 1-2 days, at temperatures that may range between 30°C and 60°C, typically between 40°C and 50 °C.

### STEP (ii); formation of the composite colloidal crystal

Precursor(s) of the continuous phase according to the present invention may be added to the colloidal crystal as obtained in step (i) in a manner that allows the precursor(s) to permeate the interstitial spaces between the particles, preferably, close-packed spheres.

Said precursor(s) can be a liquid or a solid, containing a metallic core of the same nature as the metal oxide used in the present invention and an alcoxy part covalently attached to it and defined as a polyalcoxymetaloid, such as tetraethoxysilane and tetraisopropoxytitane or a metal polyalcoxy. Typically, the precursor(s) is a solid or liquid dissolved in one or more solvents in which one or more precursors are soluble.

A precursor can be used without a solvent (i.e., neat) if it is a liquid with a sufficiently low viscosity that it can permeate the interstitial spaces. If necessary for the liquid precursors, a solvent can be used to adjust the viscosity and rate of penetration. If a solvent is used in a precursor composition, it can be water, an alcohol, for example isopropanol or methanol, an hydroalcoholic solution or other organic solvent that is compatible with the organic polymer spheres. By this it is meant that the solvent does not dissolve the particles, but sufficiently wets them to allow for penetration throughout the interstitial spaces. The precursor composition can be added to the template by soaking the template in the composition, filtering the composition through the template. Penetration of the interstitial spaces can occur simply by gravity flow, capillary action, or through the use of pressure differentials, for example, as in vacuum-assisted percolation. Preferably, the solvent and the method of penetration are selected to allow the inorganic precursor composition to penetrate the template and substantially eliminate faults in the final structure as a result of non-wetted regions.

In the framework of the present invention, a "precursor composition" means a composition comprising a precursor or mixture of precursors suitable for forming the continuous phase of the inorganic material, with or without solvent, with or without an acid in order to control the pH, in particular the pH being between 1 and 7, preferably between 2 and 5, the acid being mineral or organic, for example HCl, H₂SO₄, HCOOH, CH₃COOH, CH₃SO₃H.

The precursor composition impregnated into the colloidal crystal template may then be converted into a hardened inorganic framework around the organic polymer particles, thereby forming a composite material. This can occur through several steps and several mechanistic pathways, depending on the type of precursor.

It will undergo hydrolysis and condensation reactions to produce an oxide and water (or alcohol), which can be further removed upon drying. This is referred to herein as a "sol-gel" process, and can optionally involve the incorporation of an acid or base catalyst (e.g., hydrochloric acid, tetrapropyl ammonium hydroxide) into the inorganic precursor composition.

This material can then be converted to the desired end-product by appropriate treatment methods.

Typically, in a sol-gel process, a metal alkoxide M-(OR)ₙ undergoes hydrolysis (forming M(OH)ₓ(OR)ₙ₋ₓ), often acid- or base-catalyzed, and condensation (forming M-O-M bonds either through dehydration or dealcoholation) to form a sol that is then converted to a gel. A sol is a suspension or dispersion of discrete colloidal particles, whereas a gel is a polymeric solid containing a fluid component, which has an internal network structure such that both the solid and fluid components are highly dispersed. The gel can then be dried and calcined at an elevated temperature to form a denser ceramic material in the framework. Typically, the conversion, including sol-gel formation and drying can occur at room temperature or upon the application of heat, e.g., a temperature of 25 °C to 1000°C, although this can depend on the precursor(s) used. The calcination is typically carried out at a temperature of 400°C to 1000°C.

A typical precursor for TiO₂ used in the present invention may be titanium tetraisopropoxide (TTIP), Titanium tetrachloride (TTC), Titanium tetrabutoxide (TTBu), Titanium diisopropoxide bisacetylacetonate (Ti(acac)₂OiPr₂), tetraethylorthotitanate (TEOT), tetramethylorthotitanate (TMOT) or tetrapropylorthotitanate (TPOT).

A typical precursor for SiO₂ used in the present invention may be tetraethyl orthosilicate (TEOS) or teramethylortho silicate (TMOS).

The precursor composition comprises a mixture of an alkoxide precursor of (a) at least a metallic core of metal having a valency between 1 and 6, and of an alkoxide precursor of (b) at least SiO₂ or SiₓO_{y}, with x and y comprised independently from another between 0.1 and 2, leading to the formation after said "sol-gel" and drying processes of the continuous phase according to the present invention.

When the continuous phase is obtained from a mixture of at least a metal oxide with metal M and at least SiO₂ or SiₓO_{y}, in particular within the framework of the second embodiment, the precursor composition may comprise a M/Si molar ratio ranging from 2 to 98, in particular ranging from 5 to 95 and more particularly from 25 to 50.

In the case M is Ti, then the precursor composition may comprise a Ti/Si molar ratio ranging from 2 to 98, in particular ranging from 5 to 95 and more particularly from 25 to 50.

For both embodiments, the pH may range from 6,5 to 1, in particular from 5 to 2, more particularly from 4 to 2.

### STEP (iii): formation of the photonic crystal

The organic polymer particles of the colloidal crystal template can be removed either simultaneously with the conversion process of the precursor as described above or subsequent thereto. Typically, the template should not be removed until the material of the framework is in a sufficient state such that the porous structure does not collapse or significantly deteriorate with respect to pore size and shape, although some shrinkage is allowed. Template removal typically is achieved by either calcination (or pyrolysis) or by extraction.

Preferably, at least about 80% by weight, more preferably, at least about 95% by weight, and most preferably, at least about 98% by weight, of the organic polymer spheres are removed from the composite material. There may, however, be carbon residue remaining within the pores as explained above. For example, as much as 15% by weight carbon can remain after calcination of the template particles in a non-oxidizing atmosphere.

However, according to the second embodiment of the invention, it is preferred that the less carbon remains after calcinations. Indeed, according to a preferred aspect of the second embodiment, the material does not pertain to inverse opals, which provide colors, but focuses on obtaining photonic crystals with high transparency.

For both embodiments, calcination may typically be carried out at a temperature of 300 °C to 1000 °C, in particular of 300 °C to 700 °C, although this depends on the spheres used as a template and on the composition of the inorganic framework that is formed. The heating can for example take place in an oven. It may further be performed with specific levels depending on the nature of the metal oxide.

According to a preferred aspect of both embodiments, calcination is used to remove the organic polymer spheres.

According to the first embodiment as defined above, calcination may be performed under inert or-oxidizing atmosphere, in particular under air.

According to the second embodiment as defined above, calcination is preferably performed under an oxidizing atmosphere in order to remove most of the organic polymer spheres as a CO₂ product.

Oxidizing atmosphere may be obtained under an air flow.

It has indeed been observed that when calcination is performed under an oxidizing atmosphere, such as under an air flow, very little carbon black is deposited inside the pores of the obtained particles and the majority of the calcined polymer is transformed into carbon dioxide. In contrast, under an inert atmosphere such as under nitrogen or argon, a fair amount of carbon black remains inside the pore, generating a slightly tinted white color.

In particular, the inventors have found that the control of the temperature gradient makes it possible to control the reduction of the diameter of the pores relative to the diameter of the PMMA spheres. According to a particular embodiment, the calcination conditions are determined in such a way that the diameter reduction rate is constant.

The obtained particles, or aggregates of particles, present a size comprised between 0.8 and 500 µm, Therefore, in order to obtain particle sizes in conformity with the claimed range, i.e. 5 to 10 µm, offering high visible light reflectance, the following further steps may be performed. The obtained particles may then be dispersed in solvent, for example in an ethanol solution, then crushed and sieved to isolate particles of lower size than 20 microns.

Thereafter, the obtained particles may be dispersed in a solvent, for example an ethanol solution, at a concentration of between 0.01 g.L⁻¹ to 1 g.L⁻¹, preferably between 0.1 and 0.20 g L⁻¹. After 24 to 48 hours, the supernatant may be removed by suction, and the obtained particles may be spinned, washed with ethanol and then dried in oven with a temperature ranging from 40 to 50 ° C. Size of the obtained particles may be between 1 and 20 microns, and even between 5 and 10 microns.

### Use of the particles for improving the surface appearance of the skin

As illustrated in the following examples, the inventors have demonstrated that the herein above described particles are particularly suitable for improving the surface appearance of the skin.

Materials prepared according to the invention allow the back-scattering of a minimal amount of visible light, and the transmission of the right amount of it in order to produce a natural effect on the skin and correct the defects.

In accordance with the present invention, soft focus effect properties include at least two optical parameters: transparency and haze. There measurements were described above and is also described in the examples.

The inorganic materials of both embodiments according to the present invention allow in particular a very blurry appearance and actually reduce the visibility of fine lines and wrinkles more pronounced, in a durable manner, while being transparent in the visible, colorless or slightly colored, to ensure a natural, non-opaque appearance, when applied to skin having relief, coloration irregularities or aging related irregularities. Camouflaging effect is thus provided by implementing the inorganic materials of both embodiments according to the present invention into a cosmetic composition applied on the skin.

Indeed, as apparent in example 2 and corresponding table 3, it comes out that the inorganic material according to the invention enables to reach interesting transparency and haze values especially for material M3 (TiO₂/SiO₂ 50:50) showing comparable transparency values as comparative silica based Sunsphere H33 and PMMA based Covabeads LH85 materials, but higher haze values than these two. These same observations have been made for cosmetic films containing 3 or 5% of the material.

Particles, especially silica based ones M5-M7 of example 2 thereafter can show a light tint that can be bluish, greenish or reddish. The coloration is due to different physical factors as the spatial arrangement of the cavities inside the material causing a diffraction of incident light shined on the material. This phenomenon is also linked with the size of the pores inside the material, the nature of the particle (SiO₂ material is known by the man skilled in the art to produce such a coloring effect due to the occurrence of an energy band gap in the visible range), and the presence of additives inside the pores as carbon black.

The present invention extends to a method as described above for reducing the visible and/or tactile irregularities of the skin, comprising applying said composition to human skin in need thereof.

### Cosmetic composition

According to one of its aspects, the present invention relates or describes a cosmetic composition comprising an inorganic material conform to the present invention, i.e. conforms to the first or the second embodiment, in a cosmetically acceptable medium, in particular in an amount ranging from 0.1 to 30% by weight with respect to the total amount of the composition, in particular ranging from 0.1 to 15%, and preferably from 0.5 to 10 % by weight.

The composition according to the invention preferably has improved soft focus effect properties.

Said cosmetic compositions comprise a physiologically acceptable medium, i.e. a non-toxic medium that can be applied to human keratinic material, especially skin, and more particularly facial skin, and which is of pleasant appearance, odor and feel.

In particular, said cosmetic composition is dedicated for conferring a masking of irregularities or imperfections in the relief of the skin having such irregularities or imperfections or of an aged skin.

Said cosmetic compositions according to the present invention may in particular be applied on an aged skin.

Said cosmetic compositions may in particular be applied on skin having imperfections, in particular irregularities related to helioderma, acne scars fine lines, dilated pores or hyperkeratinization.

Said cosmetic compositions may further comprises an anti-ageing active agent and/or an active agent that acts on greasy skin, a UV filtering agent, a depigmentation active agent or a moisturizing agent.

The cosmetic composition according to the present invention may comprise a mixture of inorganic materials conform to the present invention.

The composition of the present invention may be in any form. For example, it may be a paste, a solid, a gel, or a cream or a powder. It may be an emulsion, such as an oil-in-water or water-in-oil emulsion, a multiple emulsion, such as an oil-in-water-in-oil emulsion or a water-in-oil-in-water emulsion, or a solid, rigid or supple gel, including anhydrous gels.

The composition can also be in a form chosen from a translucent anhydrous gel and a transparent anhydrous gel. The composition of the invention may, for example, comprise an external or continuous fatty phase. The composition may be anhydrous. In another embodiment, the composition of the invention may be transparent or clear, including for example, a composition without pigments. The composition can also be a molded composition or cast as a stick or a dish. The composition in one embodiment is a solid such as a molded stick or a poured stick. The compositions of the present invention may also be in the form of a lip composition such as a lipstick or a liquid lip color, a foundation or a mascara product.

The composition of the invention can also comprise any additive usually used in the field under consideration. For example, non-encapsulated pigments, film forming agents, structuring polymers, dispersants, antioxidants, essential oils, preserving agents, fragrances, waxes, liposoluble polymers that are dispersible in the medium, fillers, neutralizing agents, cosmetic and dermatological active agents such as, for example, emollients, moisturizers, vitamins, anti-wrinkle agents, essential fatty acids, sunscreens, and mixtures thereof can be added. A non-exhaustive listing of such ingredients can be found in U.S. patent application Ser. No. 10/733,467, filed Dec. 12, 2003. Further examples of suitable additional components can be found in the other references which have been incorporated in this application, including but not limited to the applications from which this application claims priority. Still further examples of such additional ingredients may be found in the International Cosmetic Ingredient Dictionary and Handbook (9th ed. 2002).

A person skilled in the art will take care to select the optional additional additives and/or the amount thereof such that the advantageous properties of the composition according to the invention are not, or are not substantially, adversely affected by the envisaged addition.

These substances may be selected variously by the person skilled in the art in order to prepare a composition which has the desired properties, for example, consistency or texture.

These additives may be present in the composition in a proportion from 0% to 99% (such as from 0.01% to 90%) relative to the total weight of the composition and further such as from 0.1% to 50% (if present).

According to preferred embodiments, the compositions comprise at least one non-encapsulated coloring agent chosen from pigments, dyes, such as liposoluble dyes, nacreous pigments, and pearling agents.

Representative liposoluble dyes which may be used according to the present invention include Sudan Red, DC Red 17, DC Green 6, β-carotene, soybean oil, Sudan Brown, DC Yellow 11, DC Violet 2, DC Orange 5, annatto, and quinoline yellow. The liposoluble dyes, when present, generally have a concentration ranging up to 20% by weight of the total weight of the composition, such as from 0.0001% to 6%.

According to preferred embodiments of the present invention, the compositions are anhydrous. By "anhydrous," it is meant that the composition contains substantially no water (that is, less than about 0.1% by weight of the composition of water).

According to other preferred embodiments, the compositions of the present invention further comprise water. In this embodiment, water is preferably present in an amount ranging from about 0.1 to about 70%, preferably from about 0.5 to 55%, and more preferably from about 1 to about 45% relative to the total weight of the composition. Preferably, such water-containing cosmetic compositions are lip compositions (for example, lipstick or liquid lip colors), foundations or mascaras, and are emulsions or dispersions.

The composition according to the invention can be in the form of a tinted or non-tinted dermatological composition or a care composition for the skin and/or the lips, in the form of an antisun composition or make-up-removing product in stick form. It can be used in particular as a care base for the skin, or the lips (lip balms, for protecting the lips against cold and/or sunlight and/or the wind, or care cream for the skin, the nails or the hair).

The composition of the invention may also be in the form of a colored make-up product for the skin, in particular a foundation, optionally having care or treating properties, a blusher, a face powder, an eye shadow, a concealer product, an eyeliner, a make-up product for the body; a make-up product for the lips such as a lipstick, optionally having care or treating properties.

Needless to say, the composition of the invention should be cosmetically or dermatologically acceptable, i.e., it should contain a non-toxic physiologically acceptable medium and should be able to be applied to the skin or the lips of human beings. For the purposes of the invention, the expression "cosmetically acceptable" means a composition of pleasant appearance, odor, feel and/or taste.

According to other preferred embodiments, methods of covering or hiding defects associated with skin such as imperfections, coloration irregularities or discolorations by applying compositions of the present invention to the skin in an amount sufficient to cover or hide such defects are provided.

In accordance with the three preceding preferred embodiments, the compositions of the present invention are applied topically to the desired area of the skin in an amount sufficient to treat, care for and/or make up the skin, to cover or hide defects associated with skin, skin imperfections, coloration irregularities or discolorations. The compositions may be applied to the desired area as needed, preferably once or twice daily, more preferably once daily and then preferably allowed to dry before subjecting to contact such as with clothing or other objects. The composition is preferably applied to the desired area that is dry or has been dried prior to application.

### EXAMPLES

### Example 1: Porous particles (M1 to M7)

Seven different materials were prepared. The porous particles M1 to M4 are encompassed within the scope of the first embodiment of the present invention, i.e. are suitable for being implemented with a cosmetic method for improving the soft focus.

### 1.1. Common procedure for the preparation of all M1 to M4 materials

### 1.1.1. Preparation of PMMA spheres

A 4-neck, 3-liter flask was fitted with a Teflon-blade stirrer, a condenser, an NMR tube containing a thermocouple, and a glass pipette nitrogen bubbler. All attachments were affixed to the flask with rubber stoppers where necessary. The flask was placed in a heating mantle, and the thermocouple was connected to a temperature controller.

For the 270-280 nm spheres used for M1 to M4, 200 mL of methyl methacrylate (99%) (MMA) and 1900 mL of water was added to the flask, and the mixture was mechanically stirred at 350 RPM. While stirring, nitrogen was slowly bubbled into the solution and the flask was heated to 80 °C until stable. After a stable temperature was reached, 2,2'-azobis(2methylpropionamidine) dihydrochloride (97%) initiator was dissolved in 18 mL of water and added to the MMA/water mixture. The nitrogen bubbler was then removed and replaced with a glass stopper, and the polymerization mixture was left to react for ~3 hours. The sphere suspension was then cooled and filtered through glass wool to remove any large polymer agglomerates that may have formed. The sphere synthesis typically yielded ~2.1 L of a 16 wt% suspension of PMMA spheres.

The PMMA spheres were characterized by SLS laser diffraction laser with a Malvern hydro 2000 apparatus.

10g of PMMA suspension was dissolved in 100g of water and the obtained solution was introduced in the measurement cell until obtaining an obscuration comprised between 3 and 4.

### 1.1.2. Colloidal crystal formation

The suspension was centrifuged at 1300 RPM for 3 days. After centrifugation, the water portion of the suspension was carefully removed by pipette without disturbing the ordered spheres. The remaining solid portion was left to dry for ~3 days. After drying, the template was easily broken apart by hand. Portions of the template that were difficult to break indicated incomplete drying and were left to dry further.

The obtained colloidal crystal was characterized by Scanning Tunneling Microscope and Scanning Electron Microscopy to measure the plain sphere diameter of the colloid and the pore diameter of the material of the invention.

### 1.1.3. Formation of the composite colloidal crystal

A liquid precursor was synthesized containing various ratios of TiO₂ to SiO₂.

A typical recipe for a liquid precursor containing the considered ratio of TiO₂ to SiO₂ consisted of the corresponding amount of tetraethyl orthosilicate (TEOS) added to the corresponding amount of isopropanol and HCl.

The mixture was then stirred using a magnetic stir bar for 1 min. After 1 min, the corresponding amount of titanium tetraisopropoxide (TTIP) was added dropwise with continued stirring. The solution became dark yellow in color and was stirred for ~5 min, resulting in a light-yellow coloration.

The dried template was separated into 18 mL glass scintillation vials with ~1-2 g of template in each vial. The broken PMMA template pieces were placed at the bottom of a scintillation vial, and the precursor was pipetted onto the bottom of the vial using a 3:4 mass ratio of precursor: template. The materials were left to infiltrate for 24 h. After infiltration, any liquid remaining outside of the template was removed by pipette, the vial was opened to the air, and the template was allowed to dry for 24 h.

### 1.1.4. Formation of the SiO₂-TiO₂ photonic crystals

The dried precursor/template composite was then placed in a 20-mL alumina boat and heated inside a quartz tube within a Lindberg/Blue M 800W tube furnace. The synthesis was performed under 0.6 L/min of flowing air in order to remove carbon from the samples to increase transmittance in the visible range. The materials were heated at 5 °C/min to 550 °C and allowed to dwell at that temperature for 2 h before cooling to ambient temperature. Changes to sample amounts and bed thickness produced no variations in sample quality.

### 1.2. Common procedure for the preparation of M5 to M7 materials

### 1.2.1. Preparation of the PMMA spheres

In a 3L jacketed reactor vessel equipped with a stirrer anchor type, 285 g of methyl methacrylate are loaded, and then 1.7 L of water. The stirring is started under a nitrogen stream in the reaction medium. The temperature of the jacket is raised to 90 °C. When the reaction temperature reaches 78 °C and stabilizes, the catalyst (1.5 g) is quickly introduced into solution in water. The nitrogen flow is stopped and then after almost 6 hours at 90 °C, the reaction mixture is cooled to room temperature. The suspension is then filtered through cotton and a suspension of 1.5 L containing 18% of a spherical PMMA is obtained.

### 1.2.2. Colloidal crystal formation

PMMA colloid suspension is centrifuged at 1000 tr.mn⁻¹ for a period of 72 hours until the spheres are no more dispersed in the supernatant solution. The supernatant water is removed and the colloidal crystal is isolated and dried in oven at 30 °C to obtain 270 g of crystal.

Characterization by scanning microscopy: the obtained image shows that the cavities are perfectly aligned.

### 1.2.3. Forming the composite colloidal crystal

A solution containing 6 g of tetraethylorthosilicate, 4 mL of methanol and 3 mL of deionized water is placed in a 100 mL flask equipped with a thermometer and a magnetic stirrer. 1 g of concentrated hydrochloric acid 36N is added to this solution at room temperature. After the exothermic phase, the reaction medium is stirred until the temperature reaches 25 °C.

Moreover, 15 g of colloidal crystal are arranged on a filter paper on a Buchner funnel. The silica precursor solution is dispersed in the colloidal crystal slowly, then wrung to remove excess solution. The infiltrated solid is recovered, transferred to an oven at 30°C for 24 hours during which the polycondensation of the silica precursor is performed to yield 17 g of the composite.

### 1.2.4. Formation of the inverse opal silica

1.2.4.1. In an oven equipped with an atmosphere control, 3 g of composite as obtained at the preceding step are placed in a calcining tube wherein a stream of nitrogen is flowing. Under a nitrogen flow of 0.6 L .mn⁻¹, the temperature is raised to 500 °C at 5 °C.min⁻¹ to 500 °C, then 700 °C for 2 hours. After furnace cooling, the solid is collected to yield 0.46 g of inverse opal with a visible color. The solid is then dispersed in ethanol after having spread on a sieve of 20 microns, then passed through the sieve. The ethanol suspension containing the particles of inverse opal stands for 24 hours at room temperature. After removing the supernatant, the solid is again dispersed in 10 ml of ethanol and the suspension is allowed to settle. After 24 hours, the supernatant is removed and the solid placed in an oven at 100 C to constant weight to obtain 0.4 g of powder of very intense color.

The maximum reflection is obtained at a certain wavelength by measuring diffuse reflectance with an Integrating Sphere RT-060-SF LabSphere.

1.2.4.2. In an oven, 3g of composite as obtained at the preceding step are placed in a calcination tube wherein a stream of air is flowing. Under an air flow of 0.6 L.min⁻¹, the temperature is raised to 500 °C at 5 °C min⁻¹ to 500 °C. After furnace cooling, the solid is collected to yield 0.42 g of inverse opal with a pale visible color. The solid is then impregnated with a solution containing 5 % w/w of sucrose, and then placed in a furnace where a stream of nitrogen is flowing like in the step 1.2.4.1. After collection of the calcinated solid, the solid is then dispersed in ethanol after having spread on a sieve of 20 microns, and then passed through the sieve. The ethanol suspension containing the particles of inverse opal stands for 24 hours at room temperature. After removing the supernatant, the solid is again dispersed in 10 ml of ethanol and the suspension is allowed to settle. After 24 hours, the supernatant is removed and the solid placed in an oven at 100°C to constant weight to obtain 0.38 g of powder of very intense color.

A SEM image allows verifying the ordered structure of the material, with pores perfectly aligned and allows verifying the required pore diameter distribution, i.e. a homogeneous pore diameter.

### 1.3. Detail amounts of reagents used in the preparation of M1 to M7 materials. (M5 to M7 being outside the invention).

The reactant amounts are gathered in the following table 1.

The diameter of the PMMA spheres is to be understood as varying +/- 5nm.

**Table 1: amounts of reagents used in the preparation of M1-M7 materials.**

| | Ratio TiO₂/SiO₂ | Amount of TEOS (g) | Amount of alcohol (g) | Amount of HCl (g) | Amount of TTIP (g) | Diameter of the PMMA spheres (nm) |
|---|---|---|---|---|---|---|
| M1 **(invention)** | 5/95 | 3.05 | 2 (isopropanol) | 0.5 | 0.22 | 277 |
| M2 **(invention)** | 25/75 | 1.2 | 1 (isopropanol) | 0.5 | 0.58 | 277 |
| M3 **(invention)** | 50/50 | 1.6 | 2 (isopropanol) | 0.5 | 2.19 | 277 |
| M4 **(invention)** | 75/25 | 0.8 | 2 (isopropanol) | 0.5 | 3.27 | 277 |
| M5 | 0/100 | 6 | 3.2 (methanol) | 1 HCl | | 321 |
| | | | | 36 N | | |
| M6 | 0/100 | 6 | 3.2 (methanol) | 1 HCl | | 410 |
| | | | | 36N | | |
| M7 | 0/100 | 6 | 3.2 (methanol) | 1 HCl | | 480 |
| | | | | 36N | | |

### 1.4. Characterization of M1-M7 materials

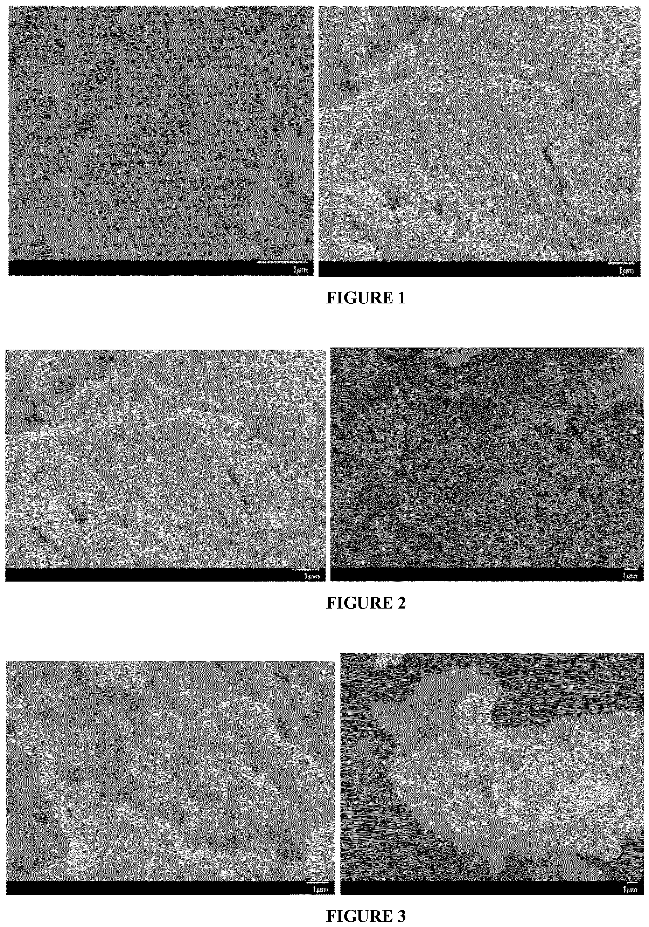

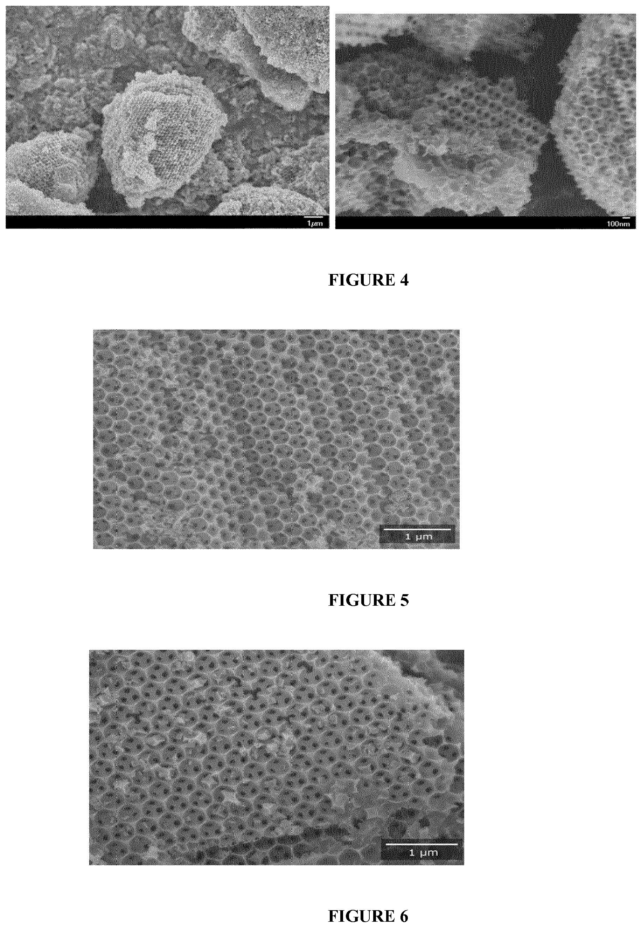

A SEM image allows verifying the ordered structure of the material, with pores perfectly aligned and allows verifying the required pore diameter distribution, i.e. a homogeneous pore diameter (see figures 1 to 7)

Moreover table 2 gathers characterization parameters for the obtained M1-M7 materials.

The size of the formed particles is constant and in the range of 5-10 µm,

The diameter of the pores is to be understood as varying +/- 5nm.

**Table 2: Characterization of the obtained materials**

| | Diameter of the pores (nm) | Pore diameter variation | Full volume fraction or % by weight of the continuous phase |
|---|---|---|---|
| M1 **(invention)** | 227 | 5 | 0,18 |
| M2 **(invention)** | 229 | 3 | 0,14 |
| M3 **(invention)** | 223 | 9 | 0,14 |
| M4 **(invention)** | 209 | 2 | 0,14 |
| M5 | 288 | 5 | 0,045 |
| M6 | 336 | 7 | 0,021 |
| M7 | 360 | 7 | 0,12 |

### Example 2: Efficiency measurement in surface appearance improvement of the skin appearance application

### 2.1. Protocol

Compositions were prepared comprising 3% by weight of each of the materials M1 to M7 (except M4) of Examples 1 by mixing the material with the base composition B1 below as reported in table 3 (3% of the material and 97% of the base B1):

### Base B1 (weight %) :

**Table 3**

| | |
|---|---|
| Cetyl alcohol | 1.25% |
| Triethanolamine | 0.375% |
| Polyethylene stearate containing 40 ethylene oxide units | 3.125% |
| 1,2-Octanediol | 0.625% |
| Hydrogenated polyisobutene (Parleam from NOF Corporation) | 6.25% |
| Isohexadecane | 18.75% |
| Mixture of glyceryl stearate and PEG-100 stearate (Arlacel^{®} 165 FL from Croda) | 3.125% |
| Carboxyvinyl polymer (Carbopol 981 polymer from Lubrizol) | 0.375% |
| Stearyl alcohol | 1.25% |
| Preserving agent | qs |
| Water | qs 100% |

Similar compositions were also prepared with the untreated silica Sunsphere H33 and Sunsphere H51 Covabeads as comparative compositions.

Each composition has been applied on a polyester thin film sold under the reference 100045425 by the society BYK, using a tire-film applicator to apply a 50 µm thick layer that was dried for 30 minutes at room temperature (25°C). On the obtained dry layer, transparency, haze and clarity measurement were performed, using a Hazegard plus apparatus from BYC Additive & Instruments.

Comparative materials are dense and non-porous PMMA Covabead particles and H33 mesoporous silica particles which cavities are not organized.

The thickness of the formed films is constant and is of 25 µm,

### 2.2. Results

Table 4 gives the transparency and haze results

**Table 4: Transparency and Haze efficiency measurement of different materials M1 to M4 obtained according to the invention**

| **Code** | **weight % w/w** | **Transparency** | **Haze** |
|---|---|---|---|
| Base Arlacel Myrj Base only **(comparative)** | | 91,52 | 5,412 |
| Sunsphere H33 Mesoporous silica which cavities are not organized (**comparative**) | 3% | 91,02 | 57,56 |
| PMMA Covabeads LH85 **(comparative)** | 3% | 92,06 | 77,68 |
| M1 **(invention)** | 3 % | 92,72 | 80,8 |
| M2 (**invenution**) | 3 % | 92,72 | 80,3 |
| M3 (**invention**) | 3% | 92,72 | 82,28 |
| M4 **(invention)** | 5% | 91,96 | 89,6 |
| M5 | 3% | 72,08 | 90,78 |
| M6 | 3% | 80,88 | 83,4 |
| M7 | 3% | 77,22 | 89,2 |

### 2.3 Conclusion

M1, M2 and M3 confer an excellent transparency in the visible range (comprised between 91 and 93) and an excellent haze power (over 80) of the cosmetic film at 3 and 5 wt%. This haze power is much superior to the porous silica material which cavities are not organized, and that is commonly used for soft focus properties (H = 57.56). It is also much more efficient than LH85 PMMA covabeads which size is centered at 10 mm and is non-porous (H = 77.68).

The comparative materials M5, M6 and M7 show an excellent power of haze and a weaker transparency compared to the comparative ones, due to the fact that they confer blue, green and red color to films containing them. Indeed, these three materials show a blue, green and red reflecting light due to the spatial arrangement of the cavities that create a diffraction phenomenon of light.

### Example 3: Cosmetic composition comprising inorganic particles M1 to M4

The compositions according to the invention as prepared in example 2 above were applied on the skin and camouflaging effect was observed on whole tested skins. In other words, the improvement of the surface appearance of the skin was clearly stated after application of said compositions to skins comprising relief, coloration irregularities or aging related irregularities of defects.

## Claims

1. A cosmetic method for improving the surface appearance of skin having relief, skin having spots or aging related irregularities, comprising applying to human skin in need thereof a composition comprising an inorganic material made of particles having a three-dimensional ordered porous structure comprising spheroidal pores,
- said pores having an average pore diameter ranging from 50 nm to 500 nm,
- the pore diameter varying by no greater than 15%,
- said particles having an average largest dimension ranging from 1 to 50 µm, and wherein
said inorganic material comprising a continuous phase being obtained from (c) a network of covalently bonded metal oxide and metalloid, the metal oxide and the metalloid being of (a) at least a metal oxide of metal having a valency between 1 and 6 and (b) at least SiO₂ or SiₓO_{y} with x and y comprised independently from another between 0.1 and 2.

2. The cosmetic method according to claim 1, wherein said method is a method for reducing the visible and/or tactile irregularities of the skin, comprising applying said composition to human skin in need thereof.

3. The cosmetic method according to claim 1 or 2, wherein the average pore diameter of the inorganic material ranges between 120 nm to 380 nm, and in particular between 150 nm to 280 nm.

4. The cosmetic method according to anyone of the preceding claims, wherein the pore diameter of the inorganic material varies by no greater than 10%, in particular by no greater than 5%.

5. The cosmetic method according to anyone of the preceding claims, wherein the average largest dimension of the particles of the inorganic material ranges from 1 to 20 µm, and in particular from 5 to 10 µm.

6. The cosmetic method according to anyone of the preceding claims, wherein the metal oxide of the particles of the inorganic material is selected from ZnO, TiO₂, Fe₂O₃, Al₂O₃ or a mixture thereof and in particular is TiO₂.

7. The cosmetic method according to anyone of the preceding claims, wherein the continuous phase is obtained from (c) a mixture of (a) TiO₂ and (b) SiO₂ or SiₓO_{y} with x and y comprised independently from another between 0.1 and 2.

8. The cosmetic method according to anyone of the preceding claims, wherein the continuous phase is present in the inorganic material in an amount ranging from 1 to 26%, in particular ranging from 3 to 10% relative to the total volume of the inorganic material.

9. The cosmetic method according to anyone of the preceding claims, wherein the inorganic material is present in the composition in an amount ranging from 0.1 to 30% by weight, in particular ranging from 0.5 to 10% by weight, relative to the total weight of the composition.

10. The cosmetic method according to anyone of the preceding claims, wherein the composition further comprises an anti-ageing active agent and/or an active agent that acts on greasy skin, a UV filtering agent, a depigmentation active agent or a moisturizing agent.

11. An inorganic material comprising particles having a three-dimensional ordered macroporous structure comprising spheroidal pores,
- said pores having an average pore diameter being greater than 280 nm and less than 500 nm,
- the pore diameter varying by no greater than 15%,
- said particles having an average largest dimension ranging from 1 to 50 µm, and wherein
said inorganic material comprising a continuous phase made of a network of covalently bonded metal oxide and metalloid, the metal oxide and the metalloid being of:
(a) at least a metal oxide of metal M having a valency between 1 and 6, and
(b) at least SiO₂ or SiₓO_{y} with x and y comprised independently from another between 0.1 and 2, in a (a)/(b) molar ratio between Si and M as referred to in (a) comprised between 5/95 and 80/20.

12. The inorganic material according to claim 11, wherein the average pore diameter of the inorganic material is greater than 280 nm and less than 380 nm.

13. The inorganic material according to claim 11 or 12, wherein the pore diameter of the inorganic material varies by no greater than 10%, in particular by no greater than 5%.

14. The inorganic material according to anyone of claims 11 to 13, wherein the metal oxide is selected from ZnO, TiO₂, Fe₂O₃, Al₂O₃ or a mixture thereof, and in particular is TiO₂.

15. The inorganic material according to anyone of claims 11 to 14, wherein the continuous phase is present in the inorganic material in an amount ranging from 1 to 26%, in particular from 3 to 10% relative to the total volume of the inorganic material.

16. A process for manufacturing an inorganic material comprising particles having a three-dimension ordered porous structure comprising a solid continuous phase and spheroidal pores comprising the steps of:
(i) providing a colloidal crystal formed of a regular array of monodisperse organic polymer particles as a template, the diameter of said organic polymer particles varying between from 65 nm and 650 nm.
(ii) adding a precursor of the continuous phase, so as to produce an amorphous solid into the interstices between the monodisperse particles of the colloidal crystal and to form said solid continuous phase and a composite colloidal crystal thereof, and
(iii) removing the monodisperse particles so as to form a regular array of pores in said solid continuous phase,
wherein said precursor of said continuous phase comprises a mixture of
(a) at least a precursor of metal oxide of metal having a valency between 1 and 6, and
(b) at least a precursor of SiO₂ or SiₓO_{y} with x and y comprised independently from another between 0.1 and 2, in a (a)/(b) molar ratio between Si and M as referred to in (a) comprised between 5/95 and 80/20.

## Patentansprüche

1. Kosmetisches Verfahren zum Verbessern des Oberflächenaussehens von Haut mit Relief, Haut mit Flecken oder altersbedingten Unregelmäßigkeiten, umfassend Aufbringen einer Zusammensetzung umfassend ein anorganisches Material aus Partikeln mit einer dreidimensionalen geordneten porösen Struktur, die sphäroidische Poren umfasst, auf menschliche Haut mit Bedarf daran,
- wobei die Poren einen mittleren Porendurchmesser in dem Bereich von 50 nm bis 500 nm aufweisen,
- wobei der Porendurchmesser um nicht mehr als 15 % variiert,
- wobei die Partikel eine mittlere größte Abmessung in dem Bereich von 1 bis 50 pm aufweisen, und wobei
das anorganische Material eine kontinuierliche Phase umfasst, die aus (c) einem Netzwerk von kovalent gebundenem Metalloxid und Metalloid erhalten ist, wobei das Metalloxid und das Metalloid aus (a) wenigstens einem Metalloxid von Metall mit einer Wertigkeit zwischen 1 und 6 und (b) wenigstens SiO₂ oder SiₓO_{y} mit x und y unabhängig voneinander in dem Bereich zwischen 0,1 und 2 bestehen.

2. Kosmetisches Verfahren gemäß Anspruch 1, wobei das Verfahren ein Verfahren zum Verringern der sichtbaren und/oder tastbaren Unregelmäßigkeiten der Haut ist, umfassend Aufbringen der Zusammensetzung auf menschliche Haut mit Bedarf daran.

3. Kosmetisches Verfahren gemäß Anspruch 1 oder 2, wobei der mittlere Porendurchmesser des anorganischen Materials in dem Bereich zwischen 120 nm und 380 nm und insbesondere zwischen 150 nm und 280 nm liegt.

4. Kosmetisches Verfahren gemäß einem der vorstehenden Ansprüche, wobei der Porendurchmesser des anorganischen Materials um nicht mehr als 10 %, insbesondere um nicht mehr als 5 %, variiert.

5. Kosmetisches Verfahren gemäß einem der vorstehenden Ansprüche, wobei die mittlere größte Abmessung der Partikel des anorganischen Materials in dem Bereich von 1 bis 20 pm und insbesondere von 5 bis 10 pm liegt.

6. Kosmetisches Verfahren gemäß einem der vorstehenden Ansprüche, wobei das Metalloxid der Partikel des anorganischen Materials aus ZnO, TiO₂, Fe₂O₃, Al₂O₃ oder einem Gemisch davon ausgewählt ist und insbesondere TiO₂ ist.

7. Kosmetisches Verfahren gemäß einem der vorstehenden Ansprüche, wobei die kontinuierliche Phase aus (c) einem Gemisch von (a) TiO₂ und (b) SiO₂ oder SiₓO_{y} mit x und y unabhängig voneinander in dem Bereich zwischen 0,1 und 2 erhalten ist.

8. Kosmetisches Verfahren gemäß einem der vorstehenden Ansprüche, wobei die kontinuierliche Phase in dem anorganischen Material in einer Menge in dem Bereich von 1 bis 26 %, insbesondere in dem Bereich von 3 bis 10 %, bezogen auf das Gesamtvolumen des anorganischen Materials vorhanden ist.

9. Kosmetisches Verfahren gemäß einem der vorstehenden Ansprüche, wobei das anorganische Material in der Zusammensetzung in einer Menge in dem Bereich von 0,1 bis 30 Gew.-%, insbesondere in dem Bereich von 0,5 bis 10 Gew.-%, bezogen auf das Gesamtgewicht der Zusammensetzung vorhanden ist.

10. Kosmetisches Verfahren gemäß einem der vorstehenden Ansprüche, wobei die Zusammensetzung ferner einen Anti-Ageing-Wirkstoff und/oder einen Wirkstoff, der auf fettige Haut wirkt, ein UV-Filtermittel, einen Entpigmentierungswirkstoff oder ein Feuchthaltemittel umfasst.

11. Anorganisches Material, umfassend Partikel mit einer dreidimensionalen geordneten makroporösen Struktur, die sphäroidische Poren umfasst,
- wobei die Poren einen mittleren Porendurchmesser aufweisen, der größer als 280 nm und kleiner als 500 nm ist,
- wobei der Porendurchmesser um nicht mehr als 15 % variiert,
- wobei die Partikel eine mittlere größte Abmessung in dem Bereich von 1 bis 50 pm aufweisen, und wobei
das anorganische Material eine kontinuierliche Phase aus einem Netzwerk von kovalent gebundenem Metalloxid und Metalloid umfasst, mit dem Metalloxid und dem Metalloid bestehend aus:
(a) wenigstens einem Metalloxid von Metall M mit einer Wertigkeit zwischen 1 und 6, und
(b) wenigstens SiO₂ oder SiₓO_{y} mit x und y unabhängig voneinander in dem Bereich zwischen 0,1 und 2 in einem (a)/(b)-Molverhältnis zwischen Si und wie in (a) bezeichnetem M in dem Bereich zwischen 5/95 und 80/20.

12. Anorganisches Material gemäß Anspruch 11, wobei der mittlere Porendurchmesser des anorganischen Materials größer als 280 nm und kleiner als 380 nm ist.

13. Anorganisches Material gemäß Anspruch 11 oder 12, wobei der Porendurchmesser des anorganischen Materials um nicht mehr als 10 %, insbesondere um nicht mehr als 5 %, variiert.

14. Anorganisches Material gemäß einem der Ansprüche 11 bis 13, wobei das Metalloxid aus ZnO, TiO₂, Fe₂O₃, Al₂O₃ oder einem Gemisch davon ausgewählt ist und insbesondere TiO₂ ist.

15. Anorganisches Material gemäß einem der Ansprüche 11 bis 14, wobei die kontinuierliche Phase in dem anorganischen Material in einer Menge in dem Bereich von 1 bis 26 %, insbesondere in dem Bereich von 3 bis 10 %, bezogen auf das Gesamtvolumen des anorganischen Materials vorhanden ist.

16. Verfahren zur Herstellung eines anorganischen Materials, das Partikel mit einer dreidimensionalen geordneten porösen Struktur umfassend eine feste kontinuierliche Phase und sphäroidische Poren umfasst, umfassend die Schritte:
(i) Bereitstellen eines kolloidalen Kristalls, gebildet aus einer regelmäßigen Anordnung von monodispersen organischen Polymerpartikeln als Templat, wobei der Durchmesser der organischen Polymerpartikel zwischen 65 nm und 650 nm variiert,
(ii) Zugeben eines Vorläufers der kontinuierlichen Phase, um einen amorphen Feststoff in den Zwischenräumen zwischen den monodispersen Partikeln des kolloidalen Kristalls zu erzeugen und um die feste kontinuierliche Phase und einen kolloidalen Verbundkristall daraus zu bilden, und
(iii) Entfernen der monodispersen Partikel, um eine regelmäßige Anordnung von Poren in der festen kontinuierlichen Phase zu bilden,
wobei der Vorläufer der kontinuierlichen Phase ein Gemisch umfasst von
(a) wenigstens einem Vorläufer von Metalloxid von Metall mit einer Wertigkeit zwischen 1 und 6, und
(b) wenigstens einem Vorläufer von SiO₂ oder SiₓO_{y} mit x und y unabhängig voneinander in dem Bereich zwischen 0,1 und 2 in einem (a)/(b)-Molverhältnis zwischen Si und wie in (a) bezeichnetem M in dem Bereich zwischen 5/95 und 80/20.

## Revendications

1. Procédé cosmétique pour l'amélioration de l'aspect de surface d'une peau ayant un relief, d'une peau ayant des taches ou des irrégularités liées au vieillissement, comprenant l'application sur une peau humaine qui en a besoin d'une composition comprenant un matériau inorganique formé de particules ayant une structure poreuse ordonnée tridimensionnelle comprenant des pores sphéroïdaux,
- lesdits pores ayant un diamètre moyen des pores allant de 50 nm à 500 nm,
- le diamètre des pores ne variant pas de plus de 15 %,
- lesdites particules ayant une plus grande dimension moyenne allant de 1 à 50 pm et
ledit matériau inorganique comprenant une phase continue qui est obtenue à partir de (c) un réseau d'oxyde métallique et de métalloïde liés de façon covalente, l'oxyde métallique et le métalloïde étant (a) au moins un oxyde métallique d'un métal ayant une valence comprise entre 1 et 6 et (b) au moins SiO₂ ou SiₓO_{y}, x et y étant compris indépendamment l'un de l'autre entre 0,1 et 2.

2. Procédé cosmétique selon la revendication 1, ledit procédé étant un procédé pour la réduction des irrégularités visibles et/ou tactiles de la peau, comprenant l'application de ladite composition sur une peau humaine qui en a besoin.

3. Procédé cosmétique selon la revendication 1 ou 2, dans lequel le diamètre moyen des pores du matériau inorganique va de 120 nm à 380 nm et en particulier de 150 nm à 280 nm.

4. Procédé cosmétique selon l'une quelconque des revendications précédentes, dans lequel le diamètre des pores du matériau inorganique ne varie pas de plus de 10 %, en particulier pas de plus de 5 %.

5. Procédé cosmétique selon l'une quelconque des revendications précédentes, dans lequel la plus grande dimension moyenne des particules du matériau inorganique va de 1 à 20 pm et en particulier de 5 à 10 pm.

6. Procédé cosmétique selon l'une quelconque des revendications précédentes, dans lequel l'oxyde métallique des particules du matériau inorganique est choisi parmi ZnO, TiO₂, Fe₂O₃, Al₂O₃ ou un mélange de ceux-ci et en particulier est TiO₂.

7. Procédé cosmétique selon l'une quelconque des revendications précédentes, dans lequel la phase continue est obtenue à partir de (c) un mélange de (a) TiO₂ et (b) SiO₂ ou SiₓO_{y}, x et y étant compris indépendamment l'un de l'autre entre 0,1 et 2.

8. Procédé cosmétique selon l'une quelconque des revendications précédentes, dans lequel la phase continue est présente dans le matériau inorganique en une quantité allant de 1 à 26 %, en particulier allant de 3 à 10 % par rapport au volume total du matériau inorganique.

9. Procédé cosmétique selon l'une quelconque des revendications précédentes, dans lequel le matériau inorganique est présent dans la composition en une quantité allant de 0,1 à 30 % en poids, en particulier allant de 0,5 à 10 % en poids, par rapport au poids total de la composition.

10. Procédé cosmétique selon l'une quelconque des revendications précédentes, dans lequel la composition comprend en outre un agent actif anti-âge et/ou un agent actif qui agit sur une peau grasse, un agent filtrant les UV, un agent actif de dépigmentation ou un agent hydratant.

11. Matériau inorganique comprenant des particules ayant une structure macroporeuse ordonnée tridimensionnelle comprenant des pores sphéroïdaux,
- lesdits pores ayant un diamètre moyen des pores qui est supérieur à 280 nm et inférieur à 500 nm,
- le diamètre des pores ne variant pas de plus de 15 %,
- lesdites particules ayant une plus grande dimension moyenne allant de 1 à 50 pm et
ledit matériau inorganique comprenant une phase continue formée d'un réseau d'oxyde métallique et de métalloïde liés de façon covalente, l'oxyde métallique et le métalloïde étant :
(a) au moins un oxyde métallique d'un métal M ayant une valence comprise entre 1 et 6 et
(b) au moins SiO₂ ou SiₓO_{y}, x et y étant compris indépendamment l'un de l'autre entre 0,1 et 2, en un rapport molaire (a)/(b) entre Si et M tel que mentionné dans (a) compris entre 5/95 et 80/20.

12. Matériau inorganique selon la revendication 11, le diamètre moyen des pores du matériau inorganique étant supérieur à 280 nm et inférieur à 380 nm.

13. Matériau inorganique selon la revendication 11 ou 12, le diamètre des pores du matériau inorganique ne variant pas de plus de 10 %, en particulier pas de plus de 5 %.

14. Matériau inorganique selon l'une quelconque des revendications 11 à 13, dans lequel l'oxyde métallique est choisi parmi ZnO, TiO₂, Fe₂O₃, Al₂O₃ ou un mélange de ceux-ci et en particulier est TiO₂.

15. Matériau inorganique selon l'une quelconque des revendications 11 à 14, la phase continue étant présente dans le matériau inorganique en une quantité allant de 1 à 26 %, en particulier de 3 à 10 % par rapport au volume total du matériau inorganique.

16. Procédé pour la fabrication d'un matériau inorganique comprenant des particules ayant une structure poreuse ordonnée tridimensionnelle comprenant une phase continue solide et des pores sphéroïdaux, comprenant les étapes consistant à :
(i) fournir un cristal colloïdal formé d'un agencement régulier de particules monodisperses de polymère organique en tant que modèle, le diamètre desdites particules de polymère organique variant entre 65 nm et 650 nm,
(ii) ajouter un précurseur de la phase continue, de manière à produire un solide amorphe dans les interstices entre les particules monodisperses du cristal colloïdal et pour former ladite phase continue solide et un cristal colloïdal composite de celle-ci et
(iii) éliminer les particules monodisperses de manière à former un agencement régulier de pores dans ladite phase continue solide,
dans lequel ledit précurseur de ladite phase continue comprend un mélange de
(a) au moins un précurseur d'oxyde métallique d'un métal ayant une valence comprise entre 1 et 6 et
(b) au moins un précurseur de SiO₂ ou de SiₓO_{y}, x et y étant compris indépendamment l'un de l'autre entre 0,1 et 2, en un rapport molaire (a)/(b) entre Si et M tel que mentionné dans (a) compris entre 5/95 et 80/20.
